(19) Europäisches Patentamt
European Patent Office
Office européen des brevets

(11) EP 0 919 535 A1

(12) DEMANDE DE BREVET EUROPEEN

(43) Date de publication:
02.06.1999 Bulletin 1999/22

(51) Int Cl.6: C07C 51/41, C07C 59/255

(21) Numéro de dépôt: 98430024.4

(22) Date de dépôt: 13.11.1998

(84) Etats contractants désignés:
AT BE CH CY DE DK ES FI FR GB GR IE IT LI LU MC NL PT SE
Etats d'extension désignés:
AL LT LV MK RO SI

(30) Priorité: 28.11.1997 FR 9715251

(71) Demandeur: Etablissements Faure et Fils
84830 Serignan du Comtat (FR)

(72) Inventeur: Faure, Jean-Pierre
84830 Serignan du Comtat (FR)

(74) Mandataire: Somnier, Jean-Louis et al
c/o Cabinet Beau de Loménie,
232, Avenue du Prado
13295 Marseille Cédex 08 (FR)

# (54) Procédé de fabrication de tartrate de calcium à partir de solution alcaline de détartrage de cuve à vin

(57) La présente invention concerne un procédé de fabrication de tartrate de calcium permettant un bon rendement industriel et fournissant un produit d'une pureté d'au moins 50%, à partir de solution alcaline de détartrage de cuve à vin tel que :

- on prépare (1) une solution alcaline de base contenant 140 à 250 grammes d'acide tartrique potentiel par litre ;
- on acidifie (2) ladite solution alcaline obtenue pour que le pH après rajout de sel de calcium soit au plus en fin de réaction égal à 8 dans le mélange réactionnel ainsi constitué ;
- on homogénéise (3) ledit mélange dans lequel on a rajouté du sel de calcium pour précipiter ledit tartrate de calcium, et on sépare (5) par tout moyen la partie solide et humide, contenant le tartrate de calcium brut, de la partie liquide qui constitue un filtrat ;
- on recycle (8) une partie au moins dudit filtrat en le rajoutant dans la préparation d'une autre solution alcaline de base contenant 140 à 250 grammes d'acide tartrique potentiel par litre.

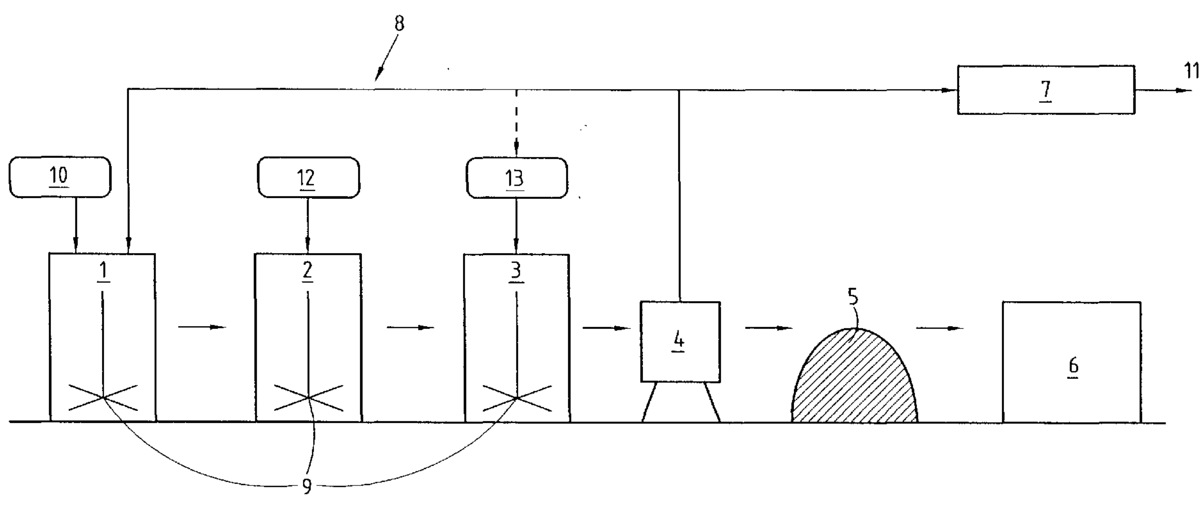




Printed by Jouve, 75001 PARIS (FR)

## Description

La présente invention a pour objet un procédé de fabrication de tartrate de calcium à partir de solution alcaline de détartrage de cuve à vin.

Le secteur technique de l'invention est le domaine de la préparation d'acides carboxyliques ou de leurs sels.

L'application principale et l'objectif de l'invention sont la valorisation des solutions alcalines de détartrage de cuve à vin afin d'en extraire du tartrate de calcium avec un bon rendement industriel rentabilisant le procédé et d'une bonne pureté afin d'intéresser les sociétés utilisatrices. En effet, le tartrate de calcium est la matière première de base pour la fabrication de l'acide tartrique.

L'acide tartrique a de nombreuses utilisations tel qu'en pharmacie (pour réaliser les produits effervescents), en vinification, dans la fabrication des jus de fruits, en biscuiterie, chocolaterie, conserve, etc... Pour satisfaire la demande, toujours croissante, il existe certes des productions d'acide tartrique synthétique mais cet acide n'est pas utilisable dans l'agro-alimentaire ; l'acide tartrique d'origine naturelle a ainsi sa part de marché d'autant plus assurée que le traitement de valorisation, tel que suivant la présente invention, permet de rendre le coût compétitif du tartrate de calcium ainsi obtenu et de fournir un produit riche en acide tartrique qui en est ensuite extrait par tous moyens et procédés connus ne faisant pas partie de la présente invention.

Le tartre est un produit de base très répandu puisqu'il constitue le dépôt cristallin que l'on retrouve sur les parois des récipients vinaires : c'est du bitartrate de potassium dont le vin est sursaturé ; il se dépose sur les parois quand la température baisse en particulier au cours de l'hiver en raison d'une moyenne de 0,2 à 0,3 kg par hectolitre de vin par an ; il constitue ainsi une matière première importante pour la fabrication de l'acide tartrique. Ce dépôt, souillé par des impuretés végétales et minérales, doit être éliminé régulièrement desdites cuves pour des raisons d'hygiène et de garantie de bonne vinification. Il y a quelques années, ce détartrage se faisait essentiellement d'une façon mécanique, en particulier dans les cuves en ciment et en bois et le tartre ainsi récupéré pouvait être directement réutilisé mais sa production est de plus en plus faible ; en effet les nouvelles cuveries sont en acier inoxydable ou en polyester ou en ciment revêtu d'enduit alimentaire ; leur détartrage ne peut s'effectuer que par voie chimique, ce qui est du reste la seule possibilité pour répondre aux exigences d'hygiène en oenologie. Cette opération est réalisée par la projection en circuit fermé d'une solution alcaline et détergente contre les parois grâce à divers dispositifs matériels connus par ailleurs, tels que ceux utilisés en laiterie, et suivant la réaction type de solubilisation suivante :

$$KHT + NaOH \Rightarrow KNaT + H_2O \text{ (avec [T] = } C_4H_4O_6\text{)}$$

le tartrate double de sodium et de potassium ainsi obtenu, appelé aussi sel de seignette est en effet soluble ; les solutions de détartrage ainsi obtenues sont alors très polluantes pour les exploitations de caves qui ne peuvent pas les rejeter dans la nature.

Ainsi, afin de pouvoir d'une part résoudre les problèmes de pollution qu'entraîne l'élimination de tels effluents, qui ne peuvent pas être rejetés en l'état en raison des contraintes actuelles de la législation et de la protection de l'environnement, et d'autre part permettre leur valorisation dans le cadre des applications indiquées en introduction, quelques chercheurs et entreprises dont le présent déposant ont travaillé sur divers procédés de traitement.

On peut citer en particulier le procédé dont est issu celui de la présente invention qui peut en être considéré comme un perfectionnement : ce procédé de base est décrit dans la demande de brevet FR 2087 232 de la société SAINT GOBAIN TECHNIQUES NOUVELLES déposée le 12 mai 1970 sur un "procédé de fabrication de tartrate de calcium". Ce document enseigne un procédé consistant à ajouter du chlorure de calcium et de l'acide chlorhydrique à une solution acqueuse contenant du carbonate de sodium ayant servi à dissoudre au moins partiellement le tartre ou gravelle laissée par le vin sur les parois des cuves. A partir de cet enseignement, différents essais ont été réalisés essentiellement en laboratoire et ont pu faire l'objet, pour certains, de publication telle que celle intitulée "les eaux résiduaires des caves de vinification" de Messieurs MOURGUES, MAUGENET, et VIGNE de l'Institut National de la Recherche Agronomique à Narbonne et publiée en décembre 1970 dans la revue des Industries Alimentaires et Agricoles N°12.

Les résultats de ces essais ont montré qu'il était effectivement possible de récupérer du tartrate de calcium et donc de l'acide tartrique à partir de solutions alcalines de détartrage de cuve à vin, mais les rendements obtenus et la teneur en acide tartrique potentiel dans le produit final étaient assez faibles et ne permettaient pas d'envisager une industrialisation à grande échelle.

Aussi, le présent déposant a étudié, réalisé des essais, et développé divers procédés spécifiques issus du procédé général ci-dessus et dont il a enfin trouvé celui décrit ci-après qui permet d'atteindre l'objectif recherché.

Il s'agit en effet d'un procédé de fabrication de tartrate de calcium tel qu'enseigné d'une manière générale par les publications ci-dessus, à partir de solution alcaline de détartrage de cuve à vin que l'on acidifie et dont on précipite ledit tartrate par ajout de sel de calcium jusqu'à obtention d'un excès de calcium. Suivant la présente invention :

- on prépare une solution alcaline de base contenant 140 à 250 grammes d'acide tartrique potentiel par litre ;
- on acidifie ladite solution alcaline obtenue pour que le pH, après rajout de sel de calcium, soit au plus en fin de réaction égal à 8 dans le mélange réactionnel ainsi constitué ;
- on homogénéise ledit mélange dans lequel on a rajouté ledit sel de calcium, on précipite ainsi le tartrate de calcium selon la réaction suivante donnée à titre d'exemple avec du chlorure de calcium :

$$KNaT + CaCl_2 \Rightarrow CaT_{\downarrow} + NaCl + KCl$$

- on sépare par tout moyen la partie solide et humide, contenant le tartrate de calcium brut, de la partie liquide qui constitue un filtrat ;
- on recycle une partie au moins dudit filtrat en le rajoutant dans la préparation d'une autre solution alcaline de base contenant 140 à 250 grammes d'acide tartrique potentiel par litre.

Selon des modes préférentiels de réalisation :

- on prépare ladite solution alcaline de base en mélangeant diverses solutions de détartrage issues d'au moins deux cuves à vin afin de pouvoir atteindre une teneur moyenne en acide tartrique qui est de préférence de l'ordre de 150 à 160 grammes par litre ; en effet, suivant l'origine des solutions alcalines de détartrage ainsi récupérées, celles-ci peuvent avoir des teneurs en acide de 80 grammes par litre jusqu'à 300 grammes par litre ;
- on recycle ledit filtrat jusqu'à ce qu'il soit saturé en sel issu de la décomposition dudit sel de calcium, soit du chlorure si on a rajouté de préférence suivant l'invention du chlorure de calcium, c'est à dire après au moins 10 cycles, et au mieux 20 ;
- on mélange ladite solution alcaline de base contenant 140 à 250 grammes d'acide tartrique par litre avec un volume quasi égal de filtrat recyclé.

Le résultat est un nouveau procédé permettant d'atteindre l'objectif recherché puisqu'en particulier on obtient en fin de traitement un produit solide qui contient entre au moins 52 à 56% d'acide tartrique potentiel, la teneur théorique maximum étant de 57,7% ; de plus grâce à la technique de recyclage on limite au maximum les effluents polluants ultimes, tout en enrichissant au mieux ledit produit solide final, permettant justement d'atteindre la concentration ci-dessus avec un rendement de plus de 95% de tartrate récupéré.

Il a pu être mesuré après 10 cycles de réutilisation des surnageants ou filtrat que le volume d'effluents résiduels était diminué de 40% par rapport au volume d'effluents recueillis après 10 opérations de traitement des solutions alcalines de mêmes caractéristiques mais sans recyclage.

De plus, en fin de recyclage de filtrat, on peut faire décanter les effluents ultimes après éventuellement un lavage pour en séparer les sels dissouts, puis, après évaporation on peut récupérer du tartrate de calcium résiduel dont les grains précipités ou cristaux trop fins n'auraient pas pu être séparés et récupérés dans la partie solide et humide et qui restent donc dans les effluents ultimes ; le reste des 5% de tartrate "perdu" par rapport au rendement de plus de 95% ci-dessus, ne peut pas être précipité et reste sous forme de tartrate double de potassium et de sodium qui est soluble. L'effluent final, non récupérable, est alors saturé en sel mais peut être ensuite traité dans une station d'épuration biologique tel que des analyses et des essais de traitabilité biologique ont pu le confirmer en laboratoire, moyennant cependant quelques précautions du fait de cette saturation en sel.

On pourrait citer d'autres avantages à la présente invention mais ceux cités en montrent déjà suffisamment pour en prouver la nouveauté et l'intérêt.

La description complémentaire ci-après et la figure jointe donnent des exemples de mise en oeuvre du procédé suivant l'invention mais n'ont aucun caractère limitatif : d'autres modes de réalisation sont possibles dans le cadre de la portée et de l'étendue de cette invention, en particulier en utilisant d'autres types d'acides ou de sels que ceux cités ci-après, mais sachant que ces derniers, à savoir, l'acide chlorhydrique et le chlorure de calcium sont ceux permettant d'obtenir un rendement optimum, tout en limitant le coût de ces produits rajoutés alors que d'autres sels et/ou d'autres acides doivent être rajoutés en plus grande quantité, ce qui influe bien entendu sur la valeur économique du produit final.

En effet, des essais et des expériences ont été réalisés et ont permis d'optimiser les résultats en utilisant en particulier et de préférence suivant l'invention :

- comme sel de calcium, du chlorure de calcium en solution à 100% mais d'autres sels de calcium pourraient être utilisés tel que du sulfate ou du carbonate, sachant qu'il faut être sûr en final que le pH du mélange réactionnel soit toujours inférieur à 8, ce qui nécessite d'une part que le pH initial soit plus faible, surtout quand le sel est

introduit non soluble dans ce mélange (voir publication citée en introduction intitulée "des eaux résiduaires des caves de vinification" de Messieurs MOURGUES, MAUGENET et VIGNE), et d'autre part de rajouter plus ou moins d'acide ;

- pour acidifier le mélange réactionnel avant l'introduction dudit sel de calcium on utilise de préférence de l'acide chlorhydrique concentré pour obtenir au plus en final ledit pH de 8 ; la quantité d'acide nécessaire en début d'opération est fonction des solutions recueillies et doit donc être contrôlée par tout homme de l'art avec tout moyen connu, sans qu'il soit nécessaire de les décrire dans la présente invention, sachant que lorsqu'on utilise du chlorure de calcium en solution, comme indiqué ci-dessus, le pH doit être maintenu entre 6,3 et 6,6 pendant la phase d'acidification et avant l'introduction de chlorure de calcium : en effet, si le pH est en dessous de 6,3, on a remarqué qu'on obtenait alors du bitartrate de potassium et non du tartrate de calcium, et si le pH est au-dessus de 6,6 on ne précipite pas alors assez celui-ci, ce qui dans tous les cas se traduit par une perte de rendement ;
- on peut également utiliser pour acidifier ledit mélange réactionnel du bitartrate de potassium solide, qui est donc en fait le tartre solide directement récupéré mécaniquement dans les cuves à vin, ou de l'acide acétique qui est également celui que l'on trouve dans le vin, ou de l'acide nitrique, ou de l'acide sulfurique ;
- le rajout du sel de calcium dans le mélange réactionnel doit être réalisé, jusqu'à observation, par un test, d'un excès de calcium dans le mélange, soit en fait de l'ordre de 5 à 10% en plus de celui strictement nécessaire ; la quantité théorique de calcium nécessaire est en effet impossible à connaître à coup sûr, a priori, car il y a une grande variabilité en fonction des lots de solutions alcalines de base, qui nécessiterait une analyse préalable de ceux-ci, comme du reste pour le rajout d'acide précédent : ceci n'est pas réalisable au stade industriel alors qu'il est plus simple de contrôler en continu la solution et de détecter par un test de présence si l'on a effectivement un excès de calcium non précipité (voir formule de la réaction précédemment donnée à titre d'exemple) dans le mélange ;
- l'homogénéisation du mélange réactionnel, après acidification et rajout de sel de calcium, est réalisée par exemple par agitation dans un réacteur de type tournant pendant 15 à 45 minutes, soit en fait de l'ordre de 30 minutes, pour obtenir la précipitation du tartrate de calcium brut ;
- la séparation dans ledit mélange réactionnel après ladite précipitation, d'une part du liquide ou filtrat, et d'autre part du produit solide humide contenant le tartrate de calcium brut que l'on veut récupérer, peut être réalisée par décantation, ou pour être plus rapide, par essorage grâce à tous équipements connus qui puissent le permettre ;
- afin d'améliorer encore la pureté ou concentration de tartrate de calcium obtenu dans ledit produit final, et d'éliminer les sels issus de la décomposition dudit sel de calcium rajouté, tels que les chlorures auxquels il est mélangé, après séparation de son filtrat, on lave la partie solide humide avec de l'eau qui dissout lesdits sels ou chlorures et on sèche le produit final ainsi obtenu ;
- si l'on veut rajouter ledit sel de calcium en solution et non sous forme solide, on peut dissoudre celui-ci, avant son introduction dans le mélange réactionnel, dans une partie du filtrat recyclé, tel que représenté par la ligne en pointillés de la figure unique jointe, entre le circuit de recyclage 8 et la réserve de chlorure de calcium 13.

Différentes expérimentations et essais ont prouvé la faisabilité du procédé suivant l'invention et permis d'atteindre l'objectif et les résultats recherchés tels que suivant l'exemple ci-dessous et en référence à la figure unique ci-jointe qui représente un schéma d'installation permettant la mise en oeuvre du procédé:

- réalisation d'un mélange initial 1 de 2.500 litres de solution alcaline de détartrage de diverses cuves à vin 10, à environ 172 grammes par litre d'acide tartrique potentiel, auxquels on rajoute et on mélange 9 2.500 litres de filtrat recyclé 8, la première opération du cycle étant réalisée avec 5.000 litres de solution alcaline de détartrage de cuves à vin 10 ;
- acidification 2 par ajout d'acide chlorhydrique 12 et agitation 9, avec mesure en continu du pH jusqu'à stabilisation entre 6,3 et 6,6 et maintien de ladite agitation 9 ;
- ajout d'environ 400 kg de chlorure de calcium 13 en solution à 100% dans l'eau jusqu'à observation d'un excès de calcium dans le mélange réactionnel (test à l'oxalate d'ammonium) avec agitation 9 maintenue, puis décantation 3 du mélange pendant une à deux heures, après arrêt de l'agitation 9 ; les trois bacs 1, 2, et 3 de traitement, représentés sur la figure avec transfert du mélange de l'un à l'autre, peuvent être remplacés par un seul bac de traitement ;
- séparation 4 du liquide et du solide obtenu par la précipitation des composés lors de la dernière réaction chimique décrite précédemment, et récupération du filtrat qui est en partie réutilisé 8 en tête de l'installation au début du procédé ;
- extraction 5 et séchage 6 au four du tartrate de calcium brut correspondant audit solide et contenant, tel que des mesures l'ont vérifié, en moyenne 54% d'acide tartrique potentiel, puis conditionnement ;
- après 10 recyclages minimum, (au cours desquels il a pu être rajouté à chaque cycle 110 à 250 litres d'acide chlorhydrique 12 suivant les lots des solutions alcalines de base 10 utilisées, ce qui montre l'importance d'un

contrôle précis du pH pour qu'en fin d'acidification celui-ci soit compris entre 6,3 et 6,6) on a produit 9,3 tonnes de tartrate de calcium brut 5 à partir des 30 $m^3$ de solutions alcalines 10 et généré 36 $m^3$ d'effluents ultimes 7 (au lieu de 61 $m^3$ d'effluents ultimes qui aurait été fournis sans ces recyclages) ; ces effluents ultimes 7, non recyclés donc, sont ensuite acheminés vers des bassins d'évaporation 11.

On peut ainsi récapituler les productions obtenues avec un tel exemple d'essais suivant le procédé de l'invention :

- quantité de tartrate de calcium produit après 10 recyclages et contenant 54% d'acide tartrique potentiel : 9.300 kg, soit :
- quantité réelle d'acide tartrique produite à partir de ce tartrate de calcium produit : 5.022 kg ;
- quantité théorique d'acide tartrique potentiel pouvant être produite à partir des 30 $m^3$ de solutions alcalines de détartrage initiales contenant en moyenne 172 grammes par litre d'acide tartrique potentiel : 5.160 kg ce qui correspond à un rendement par rapport à la quantité réelle obtenue ci-dessus de 97,3% ;

En ce qui concerne les différents volumes concernés dans un tel essai, on peut relever :

- à partir d'une première solution 10 alcaline de 5.000 litres de détartrage de cuve à vin auxquels on rajoute 1.000 litres environ d'acide, de sel et d'eau en cours de cycle, on obtient 6.000 litres de solution 3, dont on extrait environ 500 litres de produit solide 5 contenant le tartrate de calcium, 2.500 litres sont recyclés 8 et 3.000 litres constituent le filtrat ultime 7 non recyclé ;
- le volume de la solution alcaline suivante 10 de base de 2.500 litres est alors mélangé aux 2.500 litres du filtrat 8 récupéré au cycle précédent et avec les mêmes volumes moyens indiqués ci-dessus en acide, sel et eau rajoutés, on obtient ainsi après 10 cycles :
- 30 $m^3$ de solutions alcalines 10 de base ont été utilisées, issues des cuves à vin, plus 11 $m^3$ environ de rajout 12 et 13 d'acide, de sel et d'eau ; on a donc extrait, à partir des 5.000 litres de produit solide 5, 9.300 kg de produit contenant le tartrate de calcium, et 36.000 litres de filtrat ultime 7 ; alors que 61.000 litres auraient été produit sans recyclage pour obtenir la même quantité de tartrate de calcium avec bien sûr plus de solutions alcalines initiales de détartrage de cuves à vin, et bien entendu un rendement plus faible en acide tartrique que celui obtenu grâce au procédé de la présente invention.

## Revendications

1. Procédé de fabrication de tartrate de calcium à partir de solution alcaline de détartrage de cuve à vin que l'on acidifie et dont on précipite ledit tartrate de calcium par ajout de sel de calcium jusqu'à obtention d'un excès de calcium caractérisé en ce que :

   - on prépare une solution alcaline de base contenant 140 à 250 grammes d'acide tartrique potentiel par litre ;
   - on acidifie ladite solution alcaline obtenue pour que le pH après rajout de sel de calcium soit au plus en fin de réaction égal à 8 dans le mélange réactionnel ainsi constitué ;
   - on homogénéise ledit mélange dans lequel on a rajouté ledit sel de calcium et on sépare par tout moyen la partie solide et humide, contenant le tartrate de calcium brut, de la partie liquide qui constitue un filtrat ;
   - on recycle au moins une partie dudit filtrat en le rajoutant dans la préparation d'une autre solution alcaline de base contenant 140 à 250 grammes d'acide tartrique par litre.

2. Procédé suivant la revendication 1 caractérisé en ce qu'on prépare ladite solution alcaline de base en mélangeant diverses solutions issues d'au moins deux cuves à vin.

3. Procédé suivant l'une quelconque des revendications 1 à 2, caractérisé en ce qu'on recycle ledit filtrat jusqu'à ce qu'il soit saturé en sel issu de la décomposition dudit sel de calcium c'est à dire après au moins 10 cycles.

4. Procédé suivant l'une quelconque des revendications 1 à 3, caractérisé en ce qu'on mélange ladite solution alcaline de base contenant 140 à 250 grammes d'acide tartrique potentiel par litre avec un volume quasi égal de filtrat.

5. Procédé suivant l'une quelconque des revendications 1 à 4, caractérisé en ce qu'on utilise comme sel de calcium du chlorure de calcium en solution à 100% et on acidifie la solution alcaline de base, préalablement au rajout dudit chlorure de calcium, pour que le pH de cette solution alcaline soit compris entre 6,3 et 6,6.

6. Procédé suivant l'une quelconque des revendications 1 à 5, caractérisé en ce qu'après séparation de son filtrat, on lave la partie solide humide contenant le tartrate de calcium avec de l'eau qui dissout les sels issus de la décomposition dudit sel de calcium et on sèche la produit ainsi obtenu.

7. Procédé suivant l'une quelconque des revendications 1 à 6, caractérisé en ce qu'en fin de recyclage du filtrat, on le fait décanter puis évaporer et on récupère le tartrate de calcium résiduel qui a sédimenté.

8. Procédé suivant l'une quelconque des revendications 1 à 7, caractérisé en ce qu'on utilise de l'acide chlorhydrique concentré pour acidifier la solution alcaline.

9. Procédé suivant l'une quelconque des revendications 1 à 7, caractérisé en ce qu'on utilise du bitartrate de potassium solide pour acidifier le mélange réactionnel.

10. Procédé suivant l'une quelconque des revendications 1 à 9, caractérisé en ce qu'on obtient un produit solide en fin de procédé qui contient entre au moins 52 à 56% d'acide tartrique potentiel.

11. Procédé suivant l'une quelconque des revendications 1 à 10, caractérisé en ce qu'on dissout le sel de calcium, avant son introduction dans le mélange réactionnel, avec une partie du filtrat recyclé.

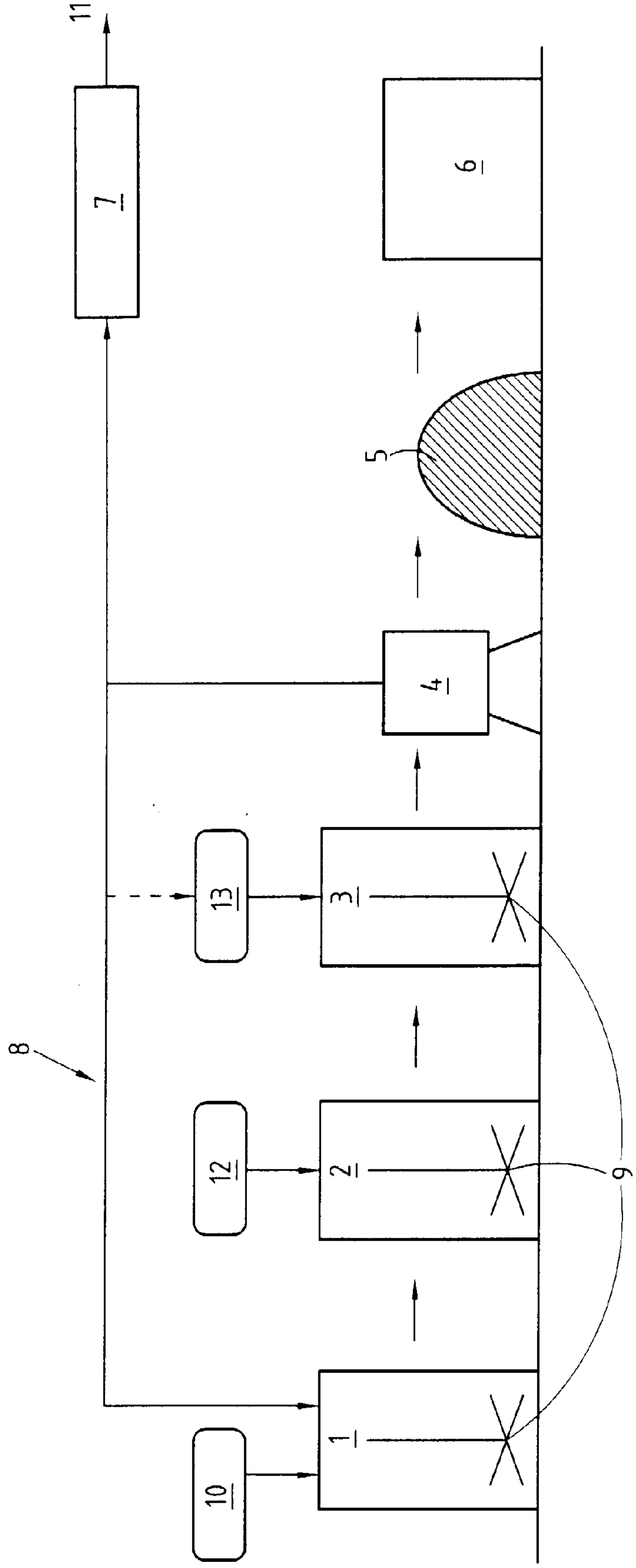
11
7
6
5
4
13
3
8
12
2
9
10
1

Office européen des brevets

# RAPPORT DE RECHERCHE EUROPEENNE

Numéro de la demande
EP 98 43 0024

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | MOURGUES, J. ; MAUGENET, J. ; VIGNE, H.: "LES EAUX RÉSIDUAIRES DES CUVES DE VINIFICATION" INDUSTRIES ALIMENTAIRES ET AGRICOLES, no. 12, décembre 1970, pages 1535-1541, XP002075125<br>* page 1536, paragraphes II.2 et II.3 *<br>* page 1538, tableau 1, essai D2 *<br>* page 1538, paragraphe d) *<br>* page 1541, première colonne, second paragraphe *<br>--- | 1 | C07C51/41<br>C07C59/255 |
| D,A | FR 2 087 232 A (SAINT GOBAIN TECHN NOUVELLES;REVICO) 31 décembre 1971<br>* page 1, ligne 1 - ligne 7 *<br>* exemple 6 *<br>* page 4, ligne 24 - ligne 27 *<br>--- | 1 | |
| A | DE 10 97 426 B (J.A. BENCKISR G.M.B.H. CHEMISCHE FABRIK) 19 janvier 1961<br>* colonne 1, ligne 17 - ligne 34 *<br>* exemple *<br>----- | 1 | **DOMAINES TECHNIQUES RECHERCHES (Int.Cl.6)**<br>C07C |

Le présent rapport a été établi pour toutes les revendications

| Lieu de la recherche | Date d'achèvement de la recherche | Examinateur |
|---|---|---|
| LA HAYE | 7 avril 1999 | Held, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 98 43 0024

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

07-04-1999

| Document brevet cité au rapport de recherche | Date de publication | Membre(s) de la famille de brevet(s) | Date de publication |
|---|---|---|---|
| FR 2087232 A | 31-12-1971 | AUCUN | |
| DE 1097426 B | | AUCUN | |

EPO FORM P0460

Pour tout renseignement concernant cette annexe : voir Journal Officiel de l'Office européen des brevets, No.12/82